(19) Europäisches Patentamt
European Patent Office
Office européen des brevets

(11) Veröffentlichungsnummer: **0 036 539**
**B1**

(12) # EUROPÄISCHE PATENTSCHRIFT

(45) Veröffentlichungstag der Patentschrift: **27.07.83**

(51) Int. Cl.³: **C 07 C 43/303,** C 07 C 41/50

(21) Anmeldenummer: **81101674.0**

(22) Anmeldetag: **07.03.81**

(54) **Verfahren zur Herstellung von Acetalen des Methylglyoxals.**

(30) Priorität: **20.03.80 DE 3010712**

(43) Veröffentlichungstag der Anmeldung: **30.09.81 Patentblatt 81/39**

(45) Bekanntmachung des Hinweises auf die Patenterteilung: **27.07.83 Patentblatt 83/30**

(84) Benannte Vertragsstaaten: **BE DE FR GB NL**

(56) Entgegenhaltungen:
**GB-A-589 877**
**US-A-3 478 060**

(73) Patentinhaber: **HOECHST AKTIENGESELLSCHAFT, Postfach 80 03 20, D-6230 Frankfurt/Main 80 (DE)**

(72) Erfinder: **Baltes, Herbert, Dr., Johannesallee 24, D-6230 Frankfurt am Main 80 (DE)**
Erfinder: **Leupold, Ernst Ingo, Dr., Am Zäunefeld 15, D-6392 Neu-Anspach (DE)**

**EP 0 036 539 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung von Acetalen des Methylglyoxals

Die Erfindung betrifft ein Verfahren zur Herstellung von Acetalen des Methylglyoxals aus Methylglyoxal und einem mit Wasser nicht oder nur begrenzt mischbaren Alkohol gemäß folgendem Schema:

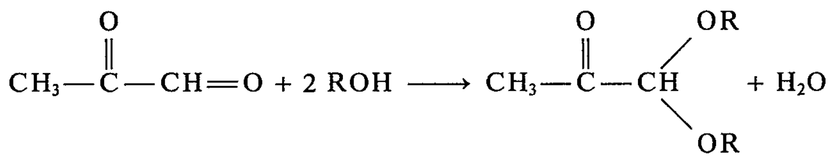

Nach US-PS 2 421 559 lassen sich Acetale des Methylglyoxals herstellen, indem man ein Gemisch aus Methylglyoxal, dem entsprechenden Alkohol ROH und einer unter 100°C siedenden, inerten, mit Wasser nicht mischbaren organischen Flüssigkeit als Schleppmittel erhitzt und das entstehende Reaktionswasser bei einer Temperatur zwischen 50°C und 100°C azeotrop abdestilliert. Als Schleppmittel werden Benzol, Diisopropylether, Ethylacetat und Ethylendichlorid angeführt; in den Beispielen wird ausschließlich Benzol verwendet. Ziel des Schleppmittel-Zusatzes ist es, das Reaktonswasser bei Temperaturen unterhalb 100°C dem Reaktionsgleichgewicht zu entziehen, da sich die Acetale des Methylglyoxals bei Temperaturen oberhalb 100°C in die Ester der $\alpha$-Alkoxycarbonsäure umlagern (US-PS 2 421 378):

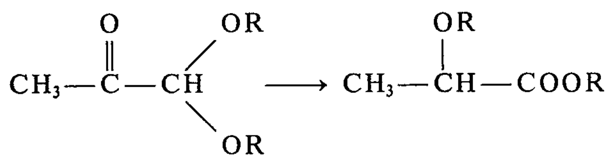

Dieses Verfahren ist wirtschaftlich und verfahrenstechnisch unbefriedigend, da der Zusatz eines Schleppmittels — abgesehen von den Materialkosten — die Aufarbeitung, insbesondere die Rückgewinnung des nicht umgesetzten Alkohols, sowie die Isolierung des Acetals beträchtlich erschwert. Einer technischen Verwendung von Benzol steht außerdem die hohe Cancerogenität im Wege.

Der Erfindung liegt die Aufgabe zugrunde, ein Verfahren zu entwickeln, das sich einerseits bei Temperaturen unterhalb 100°C durchführen läßt und somit die beschriebene Folgereaktion (Umlagerung) vermeidet und das andererseits auf den Zusatz eines an der eigentlichen Reaktion unbeteiligten Hilfsstoffes, nämlich eines Schleppmittels wie Benzol, verzichtet und somit die oben geschilderten Nachteile umgeht.

Gegenstand der vorliegenden Erfindung ist ein Verfahren zur Herstellung von Acetalen des Methylglyoxals aus Alkoholen, die mit Wasser nicht oder nur begrenzt mischbar sind, und einer wäßrigen Methylglyoxal-Lösung in Gegenwart eines sauren Katalysators unter azeotropem Entfernen des Wassers und bei Temperaturen zwischen 30°C und 100°C, das dadurch gekennzeichnet ist, daß man bei Unterdruck zwischen 0,01 und 0,8 bar das Wasser mit Hilfe des eingesetzten Alkohols als Schleppmittel azeotrop entfernt.

Das erfindungsgemäße Verfahren zeichnet sich gegenüber dem Verfahren gemäß US-PS 2 421 559 dadurch aus, daß zusätzlich zu den Reaktionskomponenten kein Hilfsstoff als Schleppmittel eingesetzt wird. Die Aufarbeitung vereinfacht sich dadurch beträchtlich. Die Ausbeuten des erfindungsgemäßen Verfahrens sind insbesondere bei der Synthese von Methylglyoxal-di-n-butylacetal deutlich höher als gemäß US-PS 2 421 559. Es war aufgrund der genannten Patentschrift nicht vorhersehbar, daß eine Reaktionsführung bei Unterdruck zwischen 0,01 und 0,8 bar einen so einfachen und wirtschaftlichen Zugang zu Acetalen des Methylglyoxals ermöglichen würde.

Das Methylglyoxal wird in wäßriger Lösung eingesetzt. Der Anteil an Methylglyoxal liegt dabei i. allg. zwischen 1 und 80 Gew.-%, vorzugsweise zwischen 20 und 60 Gew.-%. Solche wäßrigen Methylglyoxal-Lösungen fallen beispielsweise bei der Oxydehydrierung von Propylenglykol-1,2 oder Glycerin an.

Als Alkohole ROH können im Prinzip alle mit Wasser nicht oder nur begrenzt mischbaren Alkohole eingesetzt werden, insbesondere aliphatische Alkohole mit 4 bis 10 C-Atomen, beispielsweise n-Butanol, iso-Butanol, 2-Ethylhexanol-1 oder n-Octanol, vor allem aber n-Butanol.

Als saure Katalysatoren eignen sich Mineralsäuren, saure Ionenaustauscherharze oder organische Säuren; so können z. B. Schwefelsäure, Salzsäure, handelsübliche saure Ionenaustauscherharze auf Polystyrol-Basis mit $SO_3H$-Gruppen oder p-Toluolsulfonsäure eingesetzt werden. Vorzugsweise verwendet man jedoch saure Ionenaustauscherharze, da diese nach Beendigung der Reaktion problemlos abgetrennt werden können — z. B. durch Filtration — und daher eine Neutralisation und die damit verbundene Salzbildung entfallen.

Bei dem erfindungsgemäßen Verfahren setzt man pro Mol Methylglyoxal i. allg. ein:

| | | | |
|---|---|---|---|
| Alkohol ROH: | 1,2 bis 20 Mol, | vorzugsweise | 1,5 bis 10 Mol, |
| insbesondere | | | 1,5 bis 4 Mol, |
| Katalysator: | 0,01 bis 0,5 Mol, | vorzugsweise | 0,03 bis 0,2 Mol. |

Das erfindungsgemäße Verfahren wird bei Temperaturen zwischen 30°C und 100°C und einem Druck zwischen 0,01 und 0,8 bar, vorzugsweise zwischen 0,01 und 0,5 bar, durchgeführt. Eine besondere Ausführungsform des Verfahrens besteht darin, den Druck während der Reaktion so zu regeln, daß das Wasser bei einer konstanten Temperatur des Reaktionsgemisches unterhalb 100°C azeotrop abdestilliert. Der dazu benötigte Unterdruck hängt ab vom Siedepunkt des jeweiligen Alkohols.

Im einzelnen geht man i. allg. so vor, daß man in einem Rührgefäß mit Wasserabscheider ein Gemisch aus einer wäßrigen Methylglyoxal-Lösung, dem betreffenden Alkohol und einem sauren Katalysator bei Unterdruck erhitzt, bis sich keine wäßrige Phase mehr abscheidet. Der Druck wird dabei so einreguliert, daß die Temperatur des Reaktionsgemisches zu keiner Zeit 100°C übersteigt. Danach wird der Katalysator abgetrennt und das jeweilige Acetal des Methylglyoxals durch fraktionierte Destillation isoliert.

Acetale des Methylglyoxals eignen sich aufgrund ihrer hohen Reaktivität als Zwischenprodukte für die Herstellung zahlreicher chemischer Verbindungen, wie z. B. hochwirksamer Insektizide vom Allethrin-Typ (H. J. Saunders und A. W. Taff, Ind. Eng. Chem. 46, 414—426 [1954]).

## Beispiel 1

In einem 1-Liter-Dreihalskolben, ausgerüstet mit einem Rührer, einem Thermometer und einem Wasserabscheider mit Rückflußkühler, werden 150 ml 40%ige wäßrige Methylglyoxal-Lösung, 300 ml n-Butanol und 10 g eines stark sauren Ionenaustauscherharzes auf Polystyrol-Basis mit $SO_3H$-Gruppen eingebracht. Unter intensivem Rühren und bei einem Druck von 0,5 bar wird erwärmt und das Wasser azeotrop abdestilliert, bis im Wasserabscheider keine Phasentrennung mehr auftritt (ca. 6 Stunden). Die organische (obere) Phase wird in das Reaktionsgemisch zurückgeführt. Die Temperatur des Reaktionsgemisches steigt während der Umsetzung von 63°C auf ca. 95°C.

Nach Beendigung der Reaktion wird der Katalysator abfiltriert. Das nicht umgesetzte n-Butanol und Methylglyoxal werden abdestilliert und der Rückstand fraktioniert. Man erhält 159,6 g Methylglyoxal-di-n-butylacetal (Siedebereich 84°C bis 86°C/6,6 mbar), entsprechend einer Ausbeute von 79%, bezogen auf eingesetztes Methylglyoxal. $\alpha$-n-Butoxypropansäure-n-butylester läßt sich nicht nachweisen.

## Vergleichsbeispiel 1

Die Reaktion wird unter den gleichen Bedingungen wie in Beispiel 1, jedoch bei Normaldruck durchgeführt. Die Temperatur des Reaktionsgemisches steigt während der Umsetzung im Verlauf von 6 Stunden von 96°C auf ca. 124°C, die des übergehenden Dampfes von 93°C auf 117°C. Nach einer Aufarbeitung analog Beispiel 1 erhält man 148,3 g eines Gemisches aus Methylglyoxal-di-n-butylacetal (entsprechend einer Ausbeute von 60%, bezogen auf eingesetztes Methylglyoxal) und $\alpha$-n-Butoxypropansäure-n-butylester (13%). Aufgrund ähnlicher Siedepunkte (103°C bzw. 101°C bei 13 mbar) erfordert die destillative Trennung des Gemisches einen beträchtlichen Aufwand.

## Beispiel 2

In die in Beispiel 1 beschriebene Apparatur werden 300 ml 40%ige Methylglyoxal-Lösung, 500 ml n-Butanol und 30 ml wäßrige Schwefelsäure (1 n) eingebracht. Das Wasser wird wie in Beispiel 1 azeotrop entfernt. Der Druck wird im Verlauf der Reaktion von 0,4 auf 0,15 bar erniedrigt. Dadurch läßt sich eine konstante Temperatur von 75°C im Reaktionsgemisch einstellen. Nach Beendigung der Reaktion wird der Katalysator mit Natriumbutylat neutralisiert. Nach der Destillation erhält man 330,5 g Methylglyoxal-di-n-butylacetal, entsprechend einer Ausbeute von 81%.

## Beispiel 3

In die in Beispiel 1 beschriebene Apparatur werden 300 ml 40%ige Methylglyoxal-Lösung, 500 ml iso-Butanol und 15 g eines sauren Ionenaustauscherharzes auf Polystyrol-Basis mit $SO_3H$-Gruppen eingebracht. Das Wasser wird wie in Beispiel 1 bei einem Druck von 0,2 bar und einer Temperatur, die

im Versuchsverlauf von 60° C auf 85° C ansteigt, azeotrop abdestilliert. Nach der Aufarbeitung wie in Beispiel 1 erhält man 248,9 g Methylglyoxal-di-iso-butylacetal (Siedebereich 73° C bis 74° C/9,3 mbar), entsprechend einer Ausbeute von 61%.

Beispiel 4

In die in Beispiel 1 beschriebene Apparatur werden 150 ml 40%ige Methylglyoxal-Lösung, 300 ml n-Octanol und 10 g eines sauren Ionenaustauscherharzes auf Polystyrol-Basis mit $SO_3H$-Gruppen eingebracht. Das Wasser wird wie in Beispiel 1 bei einem Druck, der im Verlauf der Reaktion von 0,2 auf 0,02 bar erniedrigt wird, und einer Temperatur, die im Verlauf der Reaktion von 64° C auf 98° C ansteigt, azeotrop abdestilliert. Nach der Aufarbeitung wie in Beispiel 1 erhält man 65,7 g Methylglyoxal-di-n-octylacetal (Siedebereich 133° C bis 135° C/4 mbar), entsprechend einer Ausbeute von 65%.

**Patentanspruch**

Verfahren zur Herstellung von Acetalen des Methylglyoxals aus Alkoholen, die mit Wasser nicht oder nur begrenzt mischbar sind, und einer wäßrigen Methylglyoxal-Lösung in Gegenwart eines sauren Katalysators unter azeotropem Entfernen des Wassers und bei Temperaturen zwischen 30° C und 100° C, dadurch gekennzeichnet, daß man bei Unterdruck zwischen 0,01 und 0,8 bar das Wasser mit Hilfe des eingesetzten Alkohols als Schleppmittel azeotrop entfernt.

**Claim**

Process for the manufacture of acetals of methyl glyoxal from alcohols which are water-immiscible or water-miscible to a limited degree only and an aqueous methyl glyoxal solution, in the presence of an acidic catalyst with azeotropic removal of the water, and at temperatures of between 30° C and 100° C, characterized by removing the water as azeotropic mixture with the aid of said alcohol as entrainer at reduced pressure of between 0.01 and 0.8 bar.

**Revendication**

Procédé de préparation d'acétals du méthylglyoxal à partir d'alcools non miscibles à l'eau ou ne présentant qu'une miscibilité à l'eau limitée, et d'une solution aqueuse de méthylglyoxal, en présence d'un catalyseur acide, avec élimination azéotropique de l'eau et à des températures comprises entre 30 et 100° C, procédé caractérisé en ce qu'on élimine l'eau par distillation azéotropique sous une pression réduite comprise entre 0,01 et 0,8 bar, en utilisant, comme agent d'entraînement, l'alcool mis en jeu.